# EUROPEAN PATENT SPECIFICATION

(11) **EP 3 737 296 B1**
(45) Date of publication and mention of the grant of the patent: **24.07.2024**
(21) Application number: 18899766.2
(22) Date of filing: 20.04.2018
(51) Int. Cl.: A61B 10/02, A61B 10/00, G01N 1/02, G01N 1/28

(54) **HVS AND PAP SMEAR TESTING APPARATUS**
HVS- UND PAP-ABSTRICH-TESTVORRICHTUNG
APPAREIL DE FROTTIS HVS ET DE TEST DE PAPANICOLAOU

(30) Priority: 09.01.2018 TR 201800276
(43) Date of publication of application: 18.11.2020
(73) Proprietor: Alravvi, Omar, Fatih/Istanbul (TR)
(72) Inventor: AL-RAWE, Aeshah Omar Mahmood, Fatih/Istanbul (TR); AL-RAWE, Abu-baker Omar Mahmood, Fatih/Istanbul (TR); AL-RAWE, Othman Omar Mahmood, Fatih/Istanbul (TR); ALRAVVI, Omar, Fatih/Istanbul (TR)
(74) Representative: Yuce, Serfinaz Sibel
(86) International application number: PCT/TR2018/050182
(87) International publication number: WO 2019/139547

(56) References cited:
- WO-A1-2008/012847
- WO-A1-2010/001867
- CN-U- 205 027 584
- US-A- 2 839 049
- US-A- 4 157 709
- US-A- 4 662 381
- US-A1- 2005 277 846
- US-A1- 2005 284 239
- US-A1- 2009 105 610
- US-A1- 2011 087 133
- US-A1- 2011 105 953
- US-A1- 2016 030 020
- US-A1- 2016 262 734

## Description

### Technical Field

The subject invention is related to the HVS and Pap smear testing apparatus providing convenience for women themselves to be able to take or doctors to be able to take cervical cell sample or liquids (materials) from vagina for Human Papilloma Virus (HPV) and cultural sensitivity.

### Infrastructure of Invention

The cervix is the zone where uterus and vagina is united and contains the lower part of the uterus in the human female reproductive system. Looking at the cervix from vagina, it is in the shape of a hemisphere at the end of the vagina. The cervix is the narrow cervical area of the uterus opening to vagina. It has a very strong muscle structure. It is very tight and closed and may open only during giving birth. Cervix also helps prevent infections from reaching the uterus and serve as an important barrier.

Human Papilloma Virus (HPV) is one of the most common infections that are sexually transmitted. The HPV virus settles on penis in men and on bulb, outer sexual organs and cervix in women. When the virus HPV enters the body, it settles in the cells and is named recurrent genital wart (condyloma). Their symptoms are the warts appearing on hands and feet, respiratory tract and venereal area. It is known that there are hundreds of different sub-groups of this virus and some of these groups cause cervical cancer.

HPV infection is the most common one and seen at every age. The person contacting HPV infection has not acquired immunity to this virus. There are two reasons why the body fails to acquire immunity:
- The body is unable to respond against infection strongly and therefore the immune system of the person fails to provide long term protection. The human body may face the infection risk again for the same HPV type.
- HPV virus or the particles belonging to the virus do not mix with blood and is/are found in the cell. Therefore, it does not form an immunity to create any memory.

Cervical cancer becomes a disease with substantially high probability to recover at the stage of lesion prior to cancer or when contacted at the very early stage of it. Early diagnosis and treatment in this disease are very important. The incubation period of cervical cancer is very long. The period from appearance of lesions which are the precursor of cancer in cells to the formation of cancer varies between 5-10 years and is longer in certain cases.

Against cervical cancer, smear testing is conducted for control of the HPV infection. The purpose of this test is to know about cervical cancer and early diagnose the lesions. The smear test is known also as Papanicolaou test, Pap test, cervical smear test, cervical swab and cervical cytology. This test is carried out with routine controls since it has the effect of saving human life.

HVS test contains taking samples of vaginal secretion by means of a device similar to cotton gemma. The swab being released is placed into a special container afterwards and sent to a microbiology laboratory for a more advance analysis. Laboratory technician tries to create an artificial environment which is suitable for reproduction of bacteria. Therefore, culture and sensitivity simply means the testing process used in order to determine the necessity for definition of the bacteria in a sample and then which antibiotics to be removed. This method is used commonly in microbiology in testing various samples like various bodily discharges into urine samples for "culture and sensitivity." Obstetrician sends the sample taken directly from the liquid to microbiological analysis. Vaginal secretion is dyed and examined under microscope.

In the existing technique, Pap smear test is used as the device for collecting sample in order to be used only by doctor during gynecological examination of patient in general. For smear test, the cells discharged from cervix to vagina by means of ayre spatula or cytological brushes during gynecological examination are collected. Besides, a swab is taken form cervix by means of cytological brush. In the existing technique, since the device used for collecting sample is required to reach the squamacolumnar junction

(SCJ), namely, the area where squamous epithelium (flat) laying the outer section of cervix and columnar (cylindrical) cells laying the canal of cervix conjoin, this is difficult for collecting rod type cell. Besides, when user is using the device, since he/she will not be able place the device to SCJ zone correctly and, in case he/she inserts the device to very deep area in the body cavity when he/she tries to place the device, since there may be injuries, it is required to consult with a professional, e.g. a doctor for assistance in general. In the existing technique, the Pap smear test made by a doctor in this way is very labored and time-consuming. Besides, for women to be able to have Pap smear test to be made on a regular basis, they do not have sufficient level of access to healthcare institutions or facilities.

In the existing technique, when the sample taken from cervix is withdrawn and when being placed onto lam, there is the probability to have touched doctor or any place. Besides, in order for the sample taken not to dry or cell forms not to incur damages, the sample is required to be sent to laboratory. Thanks to the said invention, self-automatic HVS and Pap smear test apparatus that may be used by doctor is taken to laboratory environment without the risk of touching any place. It has been developed for being used automatically or by a doctor and collecting the cells readily. Especially, Pap smear search is used for taking a high vaginal swab for culture and sensitivity and early diagnosis and prevention of cervical tumor.

Subject invention ensures that patient herself may apply or a doctor may apply this test and, in this technique, there is no need to sterilize the outer genitalia completely and specially using a piece of cotton, for this reason, it is a user-friendly technique. The fact that the device has a clean lubricant ensures the entry of the device to be facilitated. Thanks to the sign of accuracy of such invention, namely, the HVS and Pap smear test apparatus, one may understand that the tissue is touched.

Such invention, namely, the HVS and Pap smear test apparatus, eliminates the disadvantages in the known condition of the technique and introduces new solutions to the problems in the existing technique.

The structural and characteristic features of the invention and all its advantages will be better understood thanks to the written explanation made below by making reference to such figures and therefore this evaluation is required to be made taking into consideration these figures and the detailed explanation.

US 4 157 709 A relates to a probe for inserting a test element into the vaginal cavity while shielding it from intermediate vaginal contact, for positioning the test element precisely in contact with the cervical os in order to collect a specimen of cervical material therefrom, and for retrieving the test element and the specimen from the vaginal cavity while shielding them from intermediate vaginal contact.

US 2005/277846 A1 relates to a cervical tissue sampling device including an exterior tube, a hold and an interior shaft.

US 2009/105610 A relates to a cell collector and cell collection method for collecting clusters of cells for subsequent analysis of the cells to screen for abnormalities

US 4 662 381 A relates to a method of collecting a sample of endometrium cells from the uterus of a subject.

US 2016/030020 A1 relates to a sample collection apparatus comprising an elongate outer casing having an insertion end and a withdrawal end.

US 2 839 049 A relates a cytologic instrument comprising a device having a holder provided with means for removing cells by abrasion from a part of a patient's body.

### Figures to assist in explanation of the invention

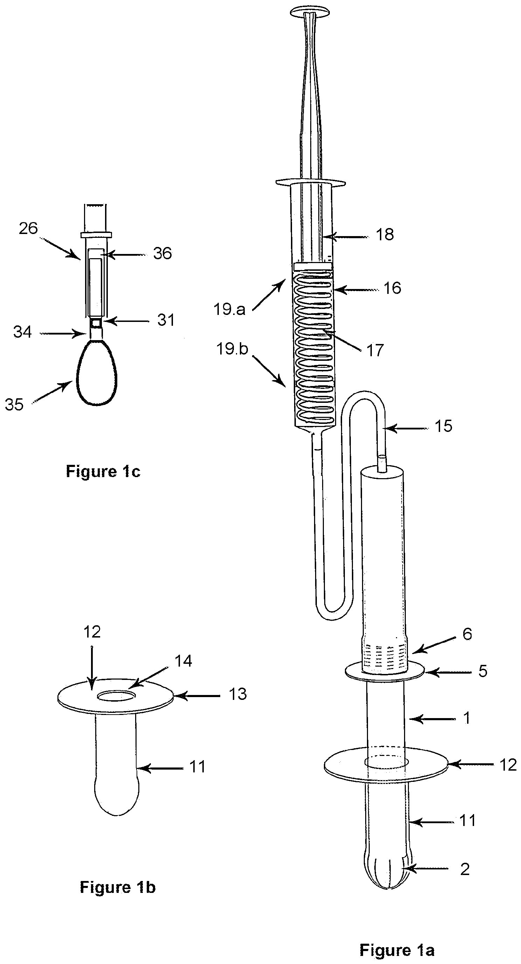

**Figure 1a****:** Outer appearance of cotton-tipped HVS and Pap smear testing apparatus used automatically
**Figure 1b****:** Outer appearance of protective cover of cotton-tipped HVS and Pap smear testing apparatus used automatically
**Figure 1c****:** Outer appearance of cotton-tip of HVS and Pap smear testing apparatus used automatically

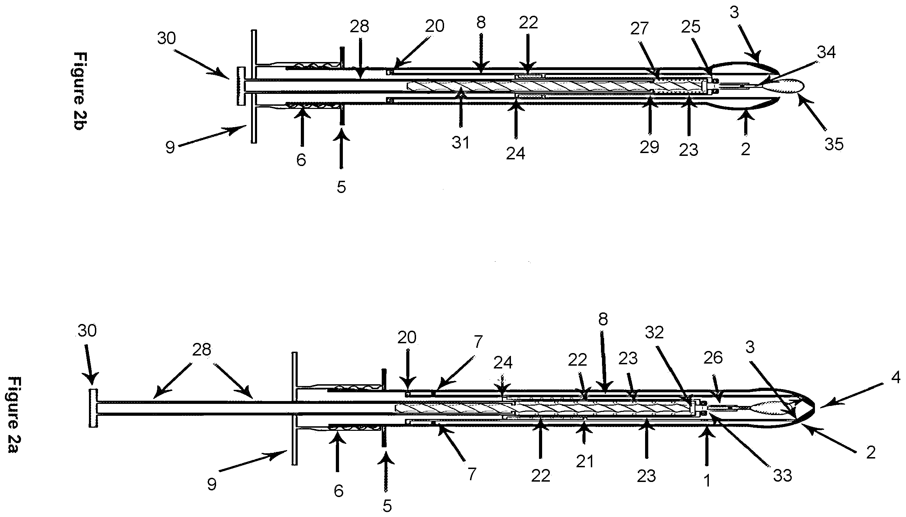

**Figure 2a****:** Side appearance of the closed condition of cotton-tipped HVS and Pap smear testing apparatus used by doctor
**Figure 2b****:** Side appearance of the open condition of cotton-tipped HVS and Pap smear testing apparatus used by doctor

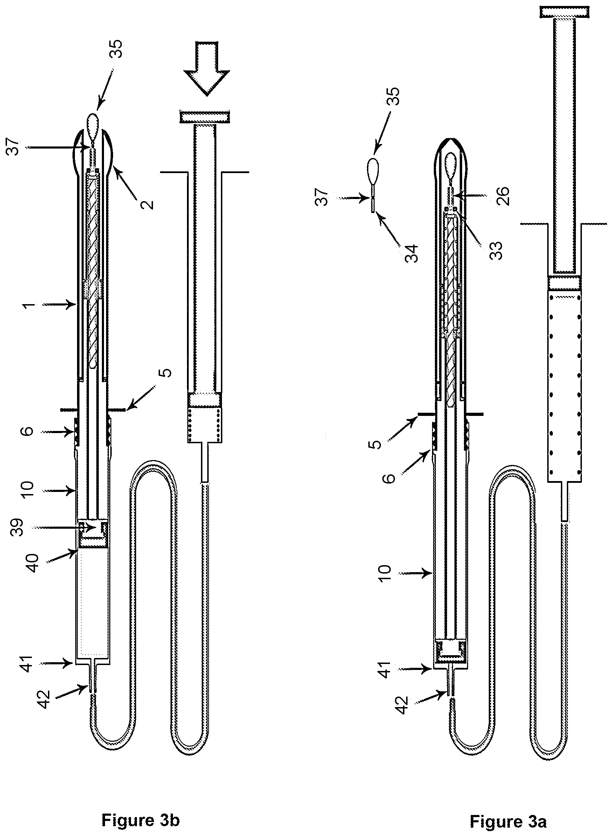

**Figure 3a****:** Side appearance of the closed condition of cotton-tipped HVS and Pap smear testing apparatus used automatically
**Figure 3b****:** Side appearance of the open condition of cotton-tipped HVS and Pap smear testing apparatus used automatically

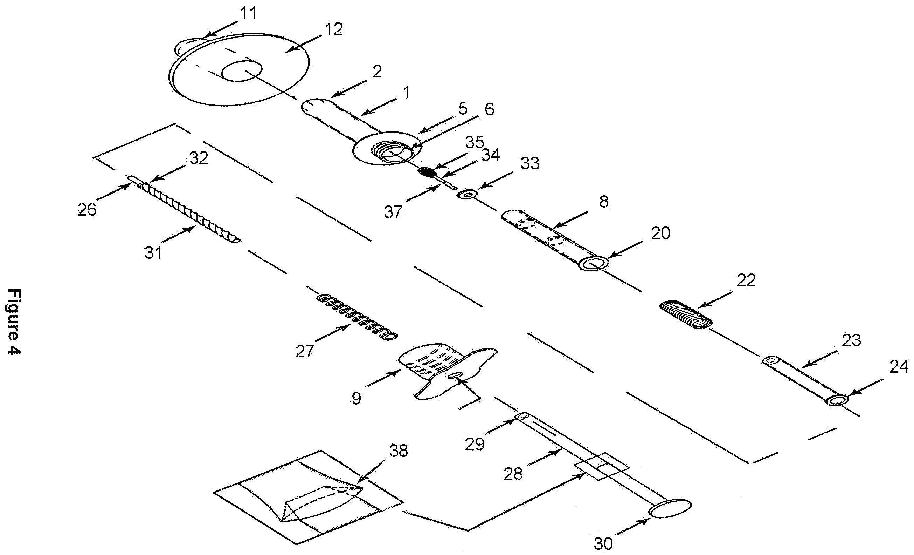

**Figure 4****:** Appearance from the top of the demounted condition of cotton-tipped HVS and Pap smear testing apparatus used by doctor

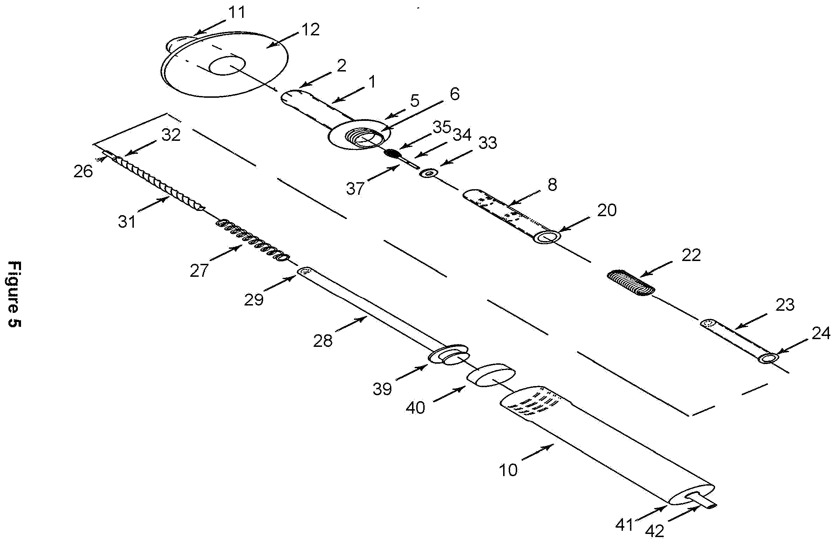

**Figure 5****:** Appearance from the top of the demounted condition of cotton-tipped HVS and Pap smear testing apparatus used automatically

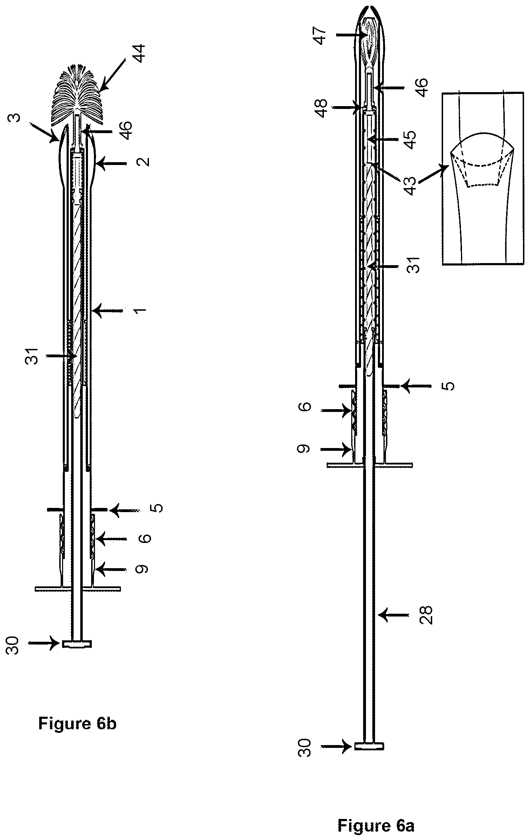

**Figure 6a****:** Side appearance of the closed condition of brush-tipped HVS and Pap smear testing apparatus used by doctor
**Figure 6b****:** Side appearance of the open condition of brush-tipped HVS and Pap smear testing apparatus used by doctor

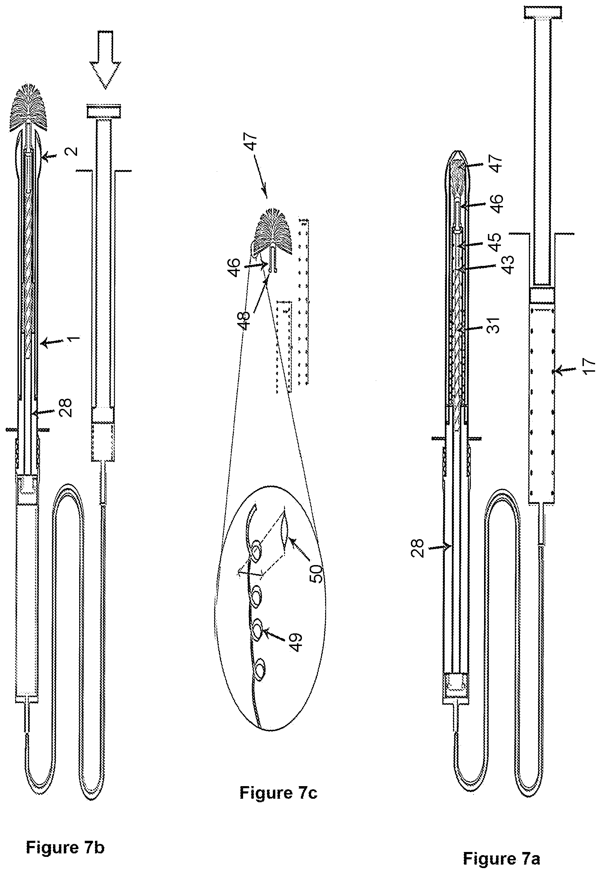

**Figure 7a****:** Side appearance of the closed condition of brush-tipped HVS and Pap smear testing apparatus used automatically
**Figure 7b****:** Side appearance of the open condition of brush-tipped HVS and Pap smear testing apparatus used automatically
**Figure 7c****:** Side appearance of the brush tip of HVS and Pap smear testing apparatus used automatically

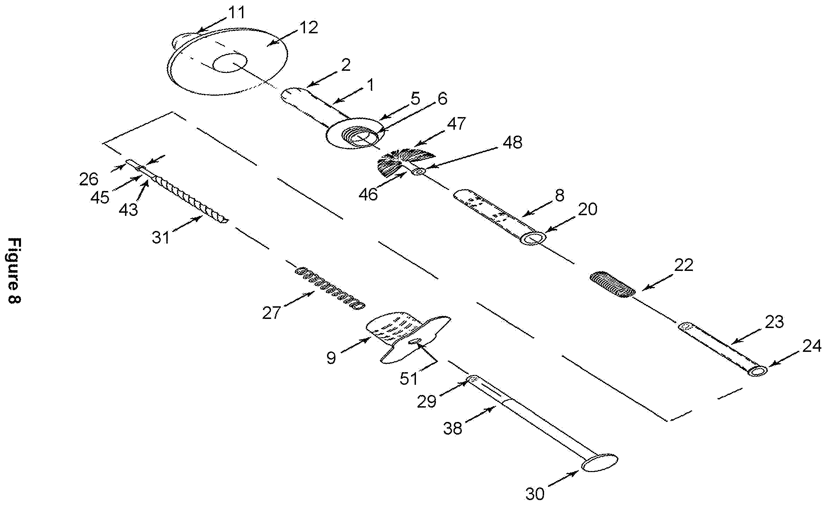

**Figure 8****:** Appearance from the top of the demounted condition of brush-tipped HVS and Pap smear testing apparatus used by doctor

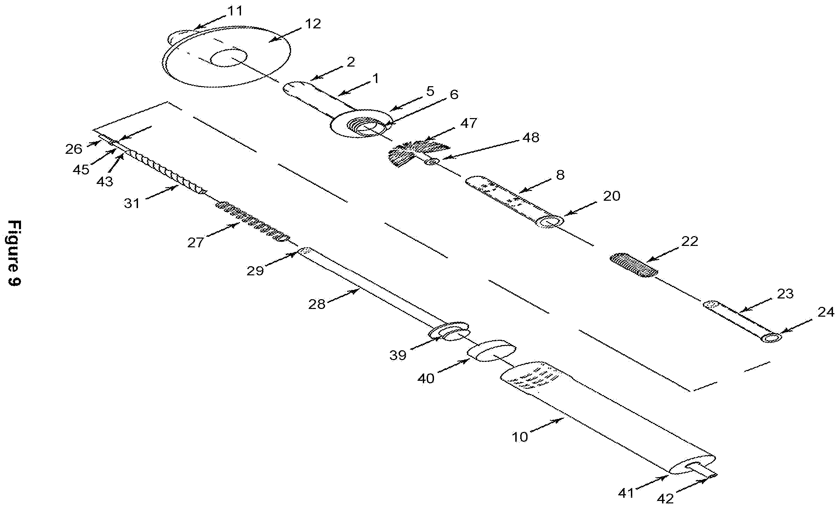

**Figure 9****:** Appearance from the top of the demounted condition of brush-tipped HVS and Pap smear testing apparatus used automatically

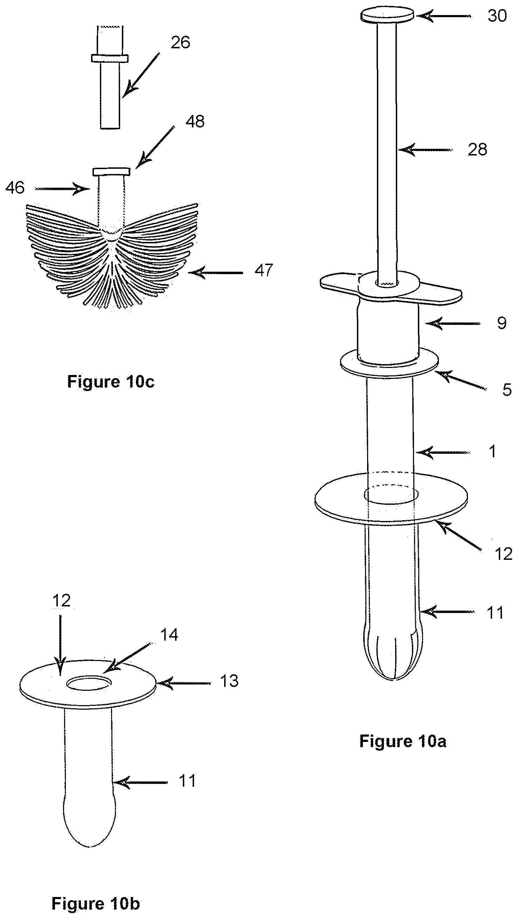

**Figure 10a****:** Outer appearance of brush-tipped HVS and Pap smear testing apparatus used by doctor
**Figure 10b****:** Outer appearance of the protective cover of brush-tipped HVS and Pap smear testing apparatus used by doctor
**Figure 10c****:** Outer appearance of brush tip of HVS and Pap smear testing apparatus used by doctor

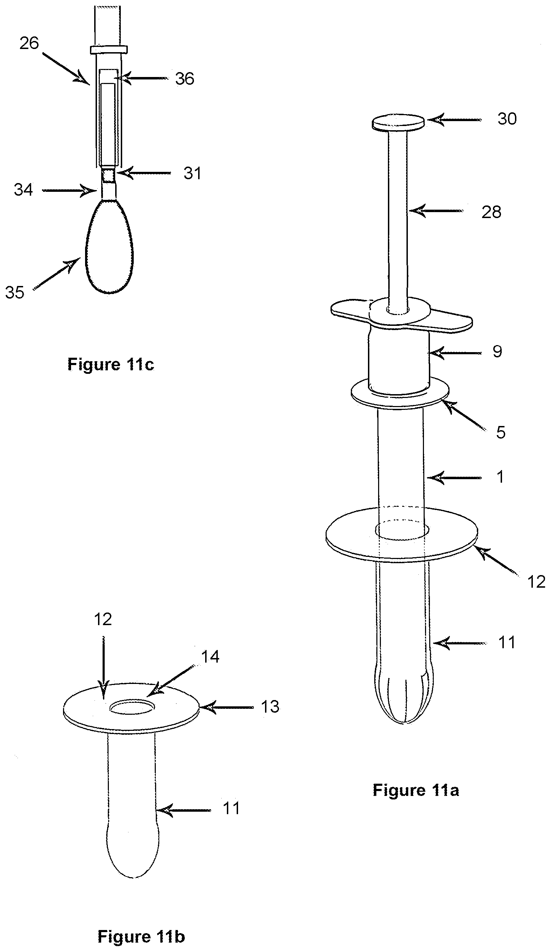

**Figure 11a****:** Outer appearance of cotton-tipped HVS and Pap smear testing apparatus used by doctor
**Figure 11b****:** Outer appearance of the protective cover of cotton-tipped HVS and Pap smear testing apparatus used by doctor
**Figure 11c****:** Outer appearance of cotton tip of HVS and Pap smear testing apparatus used by doctor

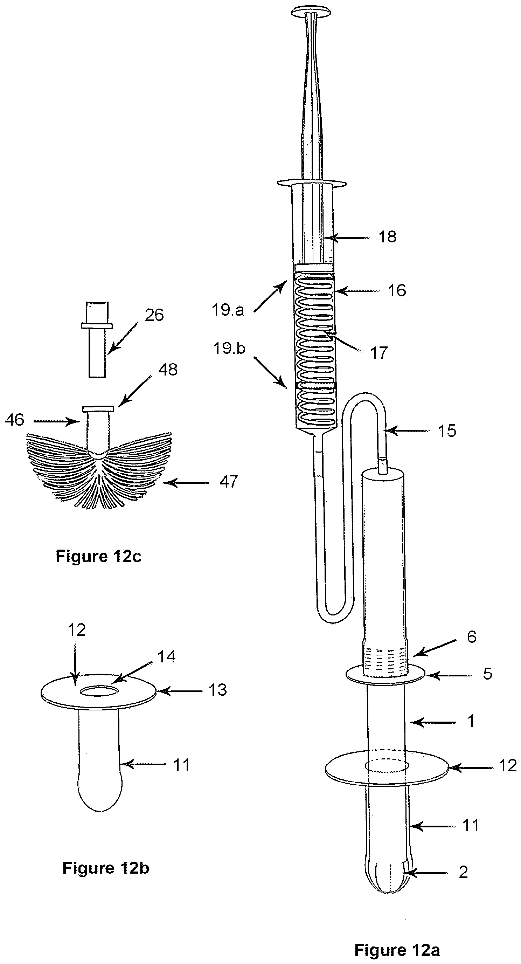

**Figure 12a****:** Outer appearance of brush-tip HVS and Pap smear testing apparatus used automatically
**Figure 12b****:** Outer appearance of the protective cover of brush-tipped HVS and Pap smear testing apparatus used automatically
**Figure 12c****:** Outer appearance of brush tip of HVS and Pap smear testing apparatus used automatically

### Reference Numbers assisting in Explanation of the Invention

**1.** Outer cylinder
**2.** Head
**3.** Interior side
**4.** End side
**5.** Ring 1
**6.** Spiral end
**7.** Ring 2a
**8.** Internal cylinder 1
**9.** Piston cover
**10.**Cylinder
**11.**Protective cover
**12.**End part
**13.**Outer end side
**14.**Interior end side
**15.**Flexible tube
**16.**Medical tube
**17.**Spring 1
**18.**Recoil cylinder 1
**19.a.** Line 1
**19.b.** Line 2
**20.** Ring 3
**21.** Ring 2b
**22.**Spring 2
**23.**Internal Cylinder 2
**24.**Ring 4
**25.**Hole 1
**26.** Tip holder
**27.**Spring 3
**28.**Recoil cylinder 2
**29.**Hole 2
**30.**Circle 1
**31.**Coil
**32.**Ring 5
**33.**Ring 6
**34.**Head bar 1
**35.**Cotton
**36.** Empty space
**37.**Accuracy sign
**38.**Cone
**39.**Circle 2
**40.**Black rubber
**41.** Rear part
**42**.Small cylinder1
**43.**Wing
**44.**Small cone
**45**.Small cylinder 2
**46**. Head bar 2
**47.**Brush
**48.**Ring 7
**49.**Brush bristle
**50.**Sharp edge
**51.** Hole 3

### Explanation of the Invention

The subject invention,which is defined by the appended claims, is characterized by the components of the HVS and Pap smear testing apparatus used by women themselves or used by doctors ensuring Pap smear test is made for Human Papilloma Virus (HPV) and HVS test for culture sensitivity. Some of the features of the system are as follows: outer cylinder (1), head (2), interior side (3), end side (4) ring 1 (5), spiral end (6), ring 2a (7), internal cylinder 1 (8), piston cover (9), cylinder (10), protective cover (11), end part (12), outer end side (13), interior end side (14), flexible tube (15), medical tube (16), spring 1 (17), recoil cylinder 1 (18), line 1 (19.a), line 2 (19.b), ring 3 (20), ring 2b (21), string 2 (22), internal cylinder 2 (23), ring 4 (24), hole 1 (25), tip holder (26), spring 3 (27), recoil cylinder 2 (28), hole 2 (29), circle 1 (30), coil (31), ring 5 (32), ring 6 (33), head bar 1 (34), cotton (35), empty space (36), accuracy sign (37), cone (38), circle 2 (39), black rubber (40), rear part (41), small cylinder 1 (42), wing (43), small cone (44), small cylinder 2 (45), head bar 2 (46), brush (47), ring 7 (48), brush bristle (49), sharp edge (50), and hole 3 (51).

The components of subject invention cotton-tipped (35) HVS and Pap smear testing apparatus used by doctor consist of:
- Outer cylinder (1) with the diameter between 1.6 and 2.4 cm and length between 10 to 14 cm
- Head part (2) intersected lengthwise in front of outer cylinder (1) and opening in rose shape,
- The thin and flat interior side (3) of the head (2) in order for heads to come out more easily and curved and thick edge part (4) for making the control,
- Ring 1 (5) with a thickness of 1 to 1,2 cm in the edge part of the outer cylinder (1),
- Spiral end (6) ensuring that outer cylinder (1) and piston cover (9) are integrated with each other,
- The ring 2a (7) and ring 2b (21) connecting the spiral end (6) to piston cover (9) and ensuring the movement of internal cylinder 1 (8),
- Internal cylinder 1 (8) moving freely within the outer cylinder (1),
- Piston cover (9) intermeshing with spiral end (6),
- Ring 3 (20) with protrusion of 1 mm in the edge part, which prevents the internal cylinder 1 (8) from moving further,
- Spring 2 (22) available between the ring 2a (7) and ring 4 (24),
- Internal cylinder 2 (23) closed from the front inside internal cylinder 1 (8); however, having a hole for head inlet in the center,
- Ring 4 (24) resting onto spring 2 (22) in the edge of internal cylinder 2 (23),
- Hole 1 (25) in the midmost ensuring the entry of the tip holder (26) in the edge part of the coil (31),
- Tip holder (26) with end side open ensuring the cotton (35) head to insert
- Spring 3 (27) available inside the internal cylinder 2 (23),
- Recoil cylinder 2 (28) ensuring the doctor give pressure to HVS and Pap smear testing apparatus,
- Hole 2 (29) in the square shape ensuring the recoil cylinder 2 (28) is connected with the coil (31) in the front side,
- Circle 1 (30) facilitating pushing with the thumb in the rear part of the recoil cylinder 2 (28),
- Cone (38) preventing the piston cover (9) from moving out again,
- Hole 3 (51) ensuring that piston cover (9) is integrated with recoil cylinder 2 (28) each other,
- Twisted coil (31) being formed in the square shape inside the recoil cylinder 2 (28),
- Ring 5 (32) between the coil (31) and tip holder (26),
- Ring 6 (33) to fix the tip holder (26),
- Head bar 1 (34) in the shape of toothpick,
- Cotton (35) in the edge of head bar 1 (34),
- Empty space (36) ensuring controlling that sample is taken with the movement of head bar 1 (34) when press applied to HVS and Pap smear testing apparatus within tip holder (26),
- The accuracy test (37) part serving to control that sample has been taken after disappearance of red line on the apparatus by the apparatus contacting the edge when pushed toward the empty space (36) after applying press to HVS and Pap smear testing apparatus.

The components of subject invention cotton-tipped (35) and brush-tipped (47) HVS and Pap smear testing apparatus used by patient automatically consist of:
- Cylinder (10) integrated with outer cylinder (1) in HVS and Pap smear testing apparatus used by patient automatically,

- Circle 2 (39) instead of circle 1 (30) differently in the edge of the recoil cylinder 2 (28),
- Black rubber (40) integrated with the circle 2 (39),
- The rear part (41) found in the edge of the cylinder (10),
- Small cylinder 1 (42) passing through the center of the rear part (41),
- Flexible tube (15) ensuring a suitable movement between cylinder (10) and medical tube (16) with a length between 25 cm to 40 cm connected to the small cylinder 1 (42),
- Medical tube (16) connected with flexible tube (15) similar to medical injector used for injection,
- Spring 1 (17) ensuring that the HVS and Pap smear testing apparatus rotates to its previous position after pressure applied by patient, which is inside the medical tube (16),
- Recoil cylinder 1 (18) ensuring that pressure is applied by patient,
- Line 1 (19.a) line 2 (19.b) controlling that the procedure has been done correctly when applied pressure by patient to recoil cylinder 1 (18), being on the medical tube (16) and sating the starting and end of the pressure applied.

The components of subject invention brush-tipped (47) HVS and Pap smear testing apparatus used by patient automatically and used by doctor consist of:
- Wing (43) ensuring that the brush-tip (47) rotates a complete tour in the edge of the coil (31),
- Small cone (44) allowing for the recoil cylinder 2 (28) to pass through the hole in one direction 2 (29),
- Small cylinder 2 (45) ensuring the further movement of the brush tip (47) in the edge of the wing (43),
- Ring 7 (48) fixing the tip holder (26), locking the system, facilitating the procedure of taking sample and ensuring rotation in the single direction,
- Head bar 2 (46) integrated with ring 7 (48),
- Brush (47) in the edge of the head bar 2 (46),
- Longer spring 2 (22) available between ring 2a (7) and ring 4 (24) for the brush (47) to rotate more,
- Longer spring 3 (27) available inside the interior cylinder 2 (23) being longer for the brush (47) to rotate more,
- Tip holder (26) with one end closed ensuring it to pass onto the brush (47),
- Spiral and transverse cross-sectioned brush (47) blister (49),
- The sharp edge (50) of the brush (47) bristle (49) ensuring that cell is taken from the cervix easily.

The components of subject invention cotton-tipped (35) and brush-tipped (47) HVS and Pap smear testing apparatus used by patient automatically and by doctor consist of:
- Protective cover (11) mountable onto the head (2) and manufactured of (latex) very thin, soft material wrapping the head, which is slippery and having a length of 4 cm,
- Curved outer edge side (13) and sloped interior edge side (14) in the edge side (12) of the protective cover (11).

The invention is used both by patient automatically and by doctor. The device provides convenience of being able to take cervical cell sample for Human Papilloma Virus (HPV) and culture sensitivity or the liquids from vagina depth. In the device, brush tip (47) is used for being able to take the cervical cell samples and cotton tip (35) for being able to liquids from vagina.

This invention consists of movable cylinder and springs and thereby when applied pressure, the head part (2) of the HVS and Pap smear testing apparatus opens and the cotton (35) or brush (47) tip takes sample from the relevant area and recedes to cylinder and closes by taking sample from the relevant area. Thus, it is prevented that the sample taken contacts anywhere. In order to be able to take sample easily from the protruding cervix, the brush (47) blisters (49) are helical and long and the edge is sharp and procures a complete tour rotation.

Subject invention reaches easily the necessary depth thank to its lubricity and the heads get out with the flexible protective cover (11) being torn easily with a simple push. When patient has realized the procedure, the invention does not harm the patient by ensuring the HVS and Pap smear testing apparatus to reach the necessary depth thanks to the line 1 (19.a) and line 2 (19.b) on the medical tube (16). After the pressure comes into existence on cotton (35) tip, the red line on the accuracy sign (37) disappears. Thus, one ensures control that sample may be taken with the accuracy sign (37).

The invention ensures that the head part (2) opens by passing force to outer cylinder (1) from flexible tube (15) by means of a push applied manually to recoil cylinder 1 (18) in HVS and Pap smear testing apparatus used automatically. The sample taken from cervix and vagina depth is procured to reach the laboratory without causing to touch anywhere.

The advantages of the invention:
- It does not give rise to any pain or discomfort in the vagina area,
- Thanks to the outer side of the head part (2) of the HVS and Pap smear testing apparatus being curved and ragged, no pain is caused when being pushed to the depths of the vagina,
- When the procedure is over in HVS and Pap smear testing apparatus, it does not require to be shut and becomes off automatically.
- When the targeted tissue is touched, the red line in accuracy sign (37) disappears, which enables confirmation feature thanks to this situation.
- HVS and Pap smear testing apparatus mounted with a single hand and directed with the other ensures control thanks to the flexible tube (15) inside the vagina,
- HVS and Pap smear testing apparatus is sterile thanks to the protective cover (11),
- HVS and Pap smear testing apparatus performs sufficient contact to cervix thanks to the automatic rotation of brush (47) tip.

The stated technique in every claim and all other features are followed by a reference number and these reference numbers have been used in order to facilitate the understanding the claims, therefore, one should not think that none of the operation steps specified with respective reference number with exemplifying purposes limit the scope.

It is obvious that a person who is specialized in the technique may also put forth the innovation in the invention by also using similar structuring and/or apply this structuring to other areas with similar purposes used in the relevant technique. Therefore, it is apparent that such structuring will be devoid of the criterion of surpassing the innovation and especially the state of the art. The scope of the invention is defined by the appended claims solely.

## Claims

1. An HVS and Pap smear testing system selectively using different tips (35, 47) and means (9+28, 10+15+16+18) for actuating sampling comprising: an outer cylinder (1), head (2) at a first end of the outer cylinder (1), an interior side (3) of the head (2), an end side (4) of the head (2), a ring 1 (5) on the outer surface and proximate the second end of the outer cylinder (1) opposite the first end, a spiral end (6) at the second end of the outer cylinder (1), a ring 2a (7) within the outer cylinder (1), an internal cylinder 1 (8), a piston cover (9), a cylinder (10), a protective cover (11) mountable onto the head (2), an end part (12) of the protective cover (11), an outer end side (13) of the end part (12), an interior end side (14) of the end part (12), a flexible tube (15) connectable to the cylinder (10), a medical tube (16) connected with the flexible tube (15), a spring 1 (17) positioned within the medical tube (16), a recoil cylinder 1 (18) slidable within the medical tube (16) to apply pressure on the spring 1 (17), a line 1 (19.a) on the medical tube (16), a line 2 (19.b) on the medical tube (16), a ring 3 (20) within the outer cylinder (1), a ring 2b (21) within the outer cylinder (1), a spring 2 (22), an internal cylinder 2 (23) within the internal cylinder 1 (8), a ring 4 (24), a hole 1 (25) at the end of the internal cylinder 2 (23), a tip holder (26), a spring 3 (27), a recoil cylinder 2 (28) slidable within the internal cylinder 2 (23), a hole 2 (29), a circle 1 (30) on a rear part of the recoil cylinder 2 (28), a coil (31), a ring 5 (32), a ring 6 (33), a head bar 1 (34) held by the tip holder (26), a cotton (35) at the edge of the head bar 1 (34), an empty space (36) within the tip holder (26), an accuracy sign (37) on the head bar 1 (34), a cone (38) on the recoil cylinder 2 (28), a circle 2 (39) positioned within cylinder (10), a black rubber (40) integrated with the circle 2 (39), a rear part (41) in the edge of the cylinder (10), a small cylinder 1 (42) passing through the center of the rear part (41) and connectable to the flexible tube (15), a wing (43) on the coil (31), a small cone (44), a small cylinder 2 (45) at the end of the coil (31), a head bar 2 (46) selectively held by the tip holder (26) when the head bar 1 (34) is not connected, a brush (47) at the edge of the head bar 2 (46), a ring 7 (48) at the other edge of the head bar 2 (46), a brush bristle (49) of the brush (47), a sharp edge (50) of the brush bristle (49), and a hole 3 (51) in the piston cover (9), wherein the testing apparatus is selectively tipped with the cotton (35) or the brush (47) as such providing the selective use of different tips, wherein
the outer cylinder (1) has a diameter between 1.6 and 2.4 cm and length between 10 to 14 cm, the head (2) intersected lengthwise in front and adapted to open in a rose shape,
the interior side (3) of the head (2) is thin and flat in order for the head (2) to open up easily and the end side (4) which is curved and thick is meant for making the control,
the ring 1 (5) has a thickness of 1 to 1.2 cm the spiral end (6) ensuring that the piston cover (9) or the cylinder (10) are selectively integrated with the outer cylinder (1) as such providing the selective use of different means for actuating sampling,
the ring 2a (7) and the ring 2b (21) connect the spiral end (6) to the piston cover (9) and ensure the movement of the internal cylinder 1 (8),
the internal cylinder 1 (8) moves freely within the outer cylinder (1),
the ring 3 (20) comprises a protrusion of 1 mm in the edge part to prevent the internal cylinder 1 (8) from moving,
the spring 2 (22) is provided between the ring 2a (7) and the ring 4 (24),
the internal cylinder 2 (23) is closed from the front inside internal cylinder 1 (8),
the ring 4 (24) rests onto the spring 2 (22) in the edge of internal cylinder 2 (23),
the hole 1 (25) is in the midmost ensuring the entry of the tip holder (26) in the edge part of the coil (31),
the tip holder (26) with the end side open ensuring the cotton (35) head to insert,
the spring 3 (27) is inside the internal cylinder 2 (23),
the recoil cylinder 2 (28) provides pressure,
the hole 2 (29) is in the square shape ensuring the recoil cylinder 2 (28) is connected with the coil (31) in the front side,
the circle 1 (30) facilitates pushing with a thumb in a rear part of the recoil cylinder 2 (28),
the cone (38) prevents the piston cover (9) from moving out,
the hole 3 (51) ensures that the piston cover (9) is integrated with the recoil cylinder 2 (28),
the coil (31) is twisted in the square shaped hole (2) inside the recoil cylinder 2 (28),
the ring 5 (32) is between the coil (31) and tip holder (26),
the ring 6 (33) fixes the tip holder (26),
the head bar 1 (34) is in a shape of toothpick,
the empty space (36) enables controlling the movement of the head bar 1 (34) to take a sample when pressure is applied within the tip holder (26), and the accuracy sign (37) indicates that a sample has been taken after disappearance of a red line on the accuracy sign (37) by the apparatus contacting the edge when pushed toward the empty space (36) after applying the pressure.

2. The HVS and Pap smear testing system selectively using different tips (35, 47) and means (9+28, 10+15+16+18) for actuating sampling as claimed in claim 1, wherein the apparatus is designed to be used by a patient and wherein the testing system is cotton tipped (37) or brushed tipped (47), and wherein the cylinder (10) is integrated with the outer cylinder (1), the flexible tube (15) ensures a movement between the cylinder (10) and the medical tube (16) with a length between 25 cm to 40 cm connected to the small cylinder 1 (42), the medical tube (16) is connected with the flexible tube (15) used for an injection, the spring 1 (17) ensuring that the testing apparatus rotates to a previous position after the pressure is applied, which is inside the medical tube (16), the recoil cylinder 1 (18) ensures that pressure is applied, the line 1 (19.a) and the line 2 (19.b) controlling that a sample has been taken correctly when applied pressure to the recoil cylinder 1 (18), being on the medical tube (16) and stating the starting and end of the pressure applied, the circle 2 (39) in the edge of the recoil cylinder 2 (28) and the black rubber (40) integrates with the circle 2 (39), the rear part (41) in the edge of the cylinder (10) and the small cylinder 1 (42) passing from the center of the rear part (41).

3. The HVS and Pap smear testing system selectively using different tips (35, 47) and means (9+28, 10+15+16+18) for actuating sampling as claimed in claim 1, wherein the system is designed to be used by patient herself or by the medical practitioner wherein the sample collector is brush tipped (47) wherein wing (43) ensuring that the brush-tip (47) rotates a complete tour in the edge of the coil (31), the small cone (44) allowing for the recoil cylinder 2 (28) to pass through the hole in one direction 2 (29), the small cylinder 2 (45) ensuring the further movement of the brush tip (47) in the edge of the wing (43), the ring 7 (48) fixing the tip holder (26), locking the system, facilitating the procedure of taking sample and ensuring rotation in the single direction, the head bar 2 (46) integrated with the ring 7 (48), the brush (47) in the edge of the head bar 2 (46), a longer spring 2 (22) available between the ring 2a (7) and the ring 4 (24) for the brush (47) to rotate more, the longer spring 3 (27) available inside the interior cylinder 2 (23) being longer for the brush (47) to rotate more, the tip holder (26) with one end closed ensuring it to pass onto the brush (47), the spiral and transverse cross-sectioned brush (47) bristle (49), wherein the sharp edge (50) of the brush (47) bristle (49) ensuring that cell is taken from the cervix easily.

4. The HVS and Pap smear testing system selectively using different tips (35, 47) and means (9+28, 10+15+16+18) for actuating sampling as claimed in claim 1, wherein the system is designed to be used by patient and by the medical practitioner wherein the sample collector is cotton tipped (35) and brush tipped (47) wherein the protective cover (11) is mountable onto the head (2) and manufactured of very thin soft material wrapping the head, which is slippery and having a length of 4 cm, the curved outer edge side (13) and the sloped interior edge side (14) in the edge side (12) of the protective cover (11).

## Patentansprüche

1. HVS- und Pap-Abstrich-Testsystem, das selektiv verschiedene Spitzen (35, 47) und Mittel (9+28, 10+15+16+18) zur Betätigung der Probenahme verwendet, umfassend: einen Außenzylinder (1), einen Kopf (2) an einem ersten Ende des Außenzylinders (1), eine Innenseite (3) des Kopfes (2), eine Endseite (4) des Kopfes (2), einen Ring (5) auf der Außenfläche und in der Nähe des zweiten Endes des Außenzylinders (1) gegenüber dem ersten Ende, ein Spiralende (6) am zweiten Ende des Außenzylinders (1), einen Ring 2a (7) innerhalb des Außenzylinders (1), einen Innenzylinder 1 (8), einen Kolbendeckel (9), einen Zylinder (10), eine Schutzabdeckung (11), die auf den Kopf (2) montiert werden kann, ein Endteil (12) der Schutzabdeckung (11), eine äußere Endseite (13) des Endteils (12), eine innere Endseite (14) des Endteils (12), ein flexibler Schlauch (15), der mit dem Zylinder (10) verbunden werden kann, einen medizinischen Schlauch (16), der mit dem flexiblen Schlauch (15) verbunden ist, eine Feder 1 (17), die in dem medizinischen Schlauch (16) angeordnet ist, einen Rückstoßzylinder 1 (18), der in dem medizinischen Schlauch (16) verschiebbar ist, um Druck auf die Feder 1 (17) auszuüben, eine Linie 1 (19. a) auf dem medizinischen Schlauch (16), eine Linie 2 (19. b) auf dem medizinischen Schlauch (16), einen Ring 3 (20) innerhalb des äußeren Zylinders (1), einen Ring 2b (21) innerhalb des äußeren Zylinders (1), eine Feder 2 (22), einen inneren Zylinder 2 (23) innerhalb des inneren Zylinders 1 (8), einen Ring 4 (24), ein Loch 1 (25) am Ende des inneren Zylinders 2 (23), einen Spitzenhalter (26), eine Feder 3 (27), einen Rückstoßzylinder 2 (28), der innerhalb des inneren Zylinders 2 (23) verschiebbar ist, ein Loch 2 (29), einen Kreis 1 (30) auf einem hinteren Teil des Rückstoßzylinders 2 (28), eine Spule (31), einen Ring 5 (32), einen Ring 6 (33), eine vom Spitzenhalter (26) gehaltene Kopfstange 1 (34), eine Watte (35) am Rand der Kopfstange 1 (34), einen leeren Raum (36) innerhalb des Spitzenhalters (26), ein Genauigkeitszeichen (37) auf der Kopfstange 1 (34), einen Kegel (38) auf dem Rückstoßzylinder 2 (28), einen Kreis 2 (39) innerhalb des Zylinders (10), ein schwarzes Gummi (40), das in den Kreis 2 (39) integriert ist, ein hinteres Teil (41) am Rand des Zylinders (10), ein kleiner Zylinder 1 (42), der durch die Mitte des hinteren Teils (41) verläuft und mit dem flexiblen Schlauch (15) verbunden werden kann, ein Flügel (43) an der Spule (31), ein kleiner Kegel (44), ein kleiner Zylinder 2 (45) am Ende der Spule (31), eine Kopfstange 2 (46), die selektiv vom Spitzenhalter (26) gehalten wird, wenn die Kopfstange 1 (34) nicht angeschlossen ist, eine Bürste (47) an der Kante der Kopfstange 2 (46), einen Ring 7 (48) an der anderen Kante der Kopfstange 2 (46), eine Bürstenborste (49) der Bürste (47), eine scharfe Kante (50) der Bürstenborste (49) und ein Loch 3 (51) in der Kolbenabdeckung (9), wobei die Prüfvorrichtung wahlweise mit der Watte (35) oder der Bürste (47) bestückt ist, wodurch die wahlweise Verwendung verschiedener Spitzen möglich ist, wobei,
der äußere Zylinder (1) hat einen Durchmesser zwischen 1,6 und 2,4 cm und eine Länge zwischen 10 und 14 cm, der Kopf (2) ist vorne in Längsrichtung gekreuzt und kann sich rosenförmig öffnen,
die Innenseite (3) des Kopfes (2) ist dünn und flach, damit sich der Kopf (2) leicht öffnen lässt, und die Endseite (4), die gekrümmt und dick ist, dient zur Herstellung der Kontrolle,
der Ring 1 (5) hat eine Dicke von 1 bis 1,2 cm, wobei das spiralförmige Ende (6) dafür sorgt, dass der Kolbendeckel (9) oder der Zylinder (10) selektiv in den Außenzylinder (1) integriert werden und so die selektive Verwendung verschiedener Mittel zur Betätigung der Probenahme ermöglicht,
der Ring 2a (7) und der Ring 2b (21) das Spiralende (6) mit dem Kolbendeckel (9) verbinden und die Bewegung des Innenzylinders 1 (8) gewährleisten, wobei sich der Innenzylinder 1 (8) frei im Außenzylinder (1) bewegt,
der Ring 3 (20) im Randbereich einen Vorsprung von 1 mm aufweist, um die Bewegung des inneren Zylinders 1 (8) zu verhindern,
die Feder 2 (22) ist zwischen dem Ring 2a (7) und dem Ring 4 (24) vorgesehen, der Innenzylinder 2 (23) ist von vorne im Innenzylinder 1 (8) geschlossen,
der Ring 4 (24) liegt auf der Feder 2 (22) im Rand des Innenzylinders 2 (23) auf,
das Loch 1 (25) befindet sich in der Mitte und gewährleistet den Eintritt des Spitzenhalters (26) in den Randbereich der Spule (31),
der Spitzenhalter (26) ist an der Endseite offen, so dass der Kopf der Watte (35) eingeführt werden kann,
die Feder 3 (27) befindet sich im inneren des Innenzylinders 2 (23),
der Rückstoßzylinder 2 (28) sorgt für Druck,
das Loch 2 (29) hat eine quadratische Form, so dass der Rückstoßzylinder 2 (28) mit der Spule (31) an der Vorderseite verbunden ist,
der Kreis 1 (30) erleichtert das Drücken mit dem Daumen in einem hinteren Teil des Rückstoßzylinders 2 (28),
der Konus (38) verhindert das Herausschieben des Kolbendeckels (9),
das Loch 3 (51) sorgt dafür, dass die Kolbenabdeckung (9) in den Rückstoßzylinder 2 (28) integriert wird Zylinder 2 (28),
die Spule (31) ist in dem quadratischen Loch (2) im inneren des Rückstoßzylinders 2 (28) verdreht,
der Ring 5 (32) befindet sich zwischen der Spule (31) und dem Spitzenhalter (26),
der Ring 6 (33) fixiert den Spitzenhalter (26),
der Kopfbalken 1 (34) hat die Form eines Zahnstochers, der leere Raum (36) ermöglicht die Steuerung der Bewegung des Kopfbalkens 1 (34), um eine Probe zu entnehmen, wenn Druck innerhalb des Spitzenhalters (26) ausgeübt wird, und das Genauigkeitszeichen (37) zeigt an, dass eine Probe entnommen wurde, nachdem eine rote Linie auf dem Genauigkeitszeichen (37) verschwunden ist, indem die Vorrichtung die Kante berührt, wenn sie nach dem Ausüben des Drucks in Richtung des leeren Raums (36) gedrückt wird.

2. HVS- und Pap-Abstrich-Testsystem, das selektiv verschiedene Spitzen (35, 47) und Mittel (9+28, 10+15+16+18) zur Betätigung der Probenahme verwendet, wie in Anspruch 1 beansprucht, wobei die Vorrichtung zur Verwendung durch einen Patienten bestimmt ist und wobei das Testsystem mit einer Baumwollspitze (37) oder einer Bürstenspitze (47) versehen ist und wobei der Zylinder (10) mit dem äußeren Zylinder (1) integriert ist, der biegsame Schlauch (15) eine Bewegung zwischen dem Zylinder (10) und dem medizinischen Schlauch (16) mit einer Länge zwischen 25 cm und 40 cm gewährleistet, der mit dem kleinen Zylinder 1 (42) verbunden ist, der medizinische Schlauch (16) mit dem biegsamen Schlauch (15) verbunden ist, der für eine Injektion verwendet wird, die Feder 1 (17) sicherstellt, dass sich die Testvorrichtung nach dem Aufbringen des Drucks in eine vorherige Position dreht, die sich innerhalb des medizinischen Schlauchs (16) befindet, der Rückstoßzylinder 1 (18) sicherstellt, dass Druck aufgebracht wird, die Leitung 1 (19. a) und die Linie 2 (19.b) kontrollieren, dass eine Probe korrekt entnommen wurde, wenn Druck auf den Rückstoßzylinder 1 (18) ausgeübt wurde, der sich auf dem medizinischen Schlauch (16) befindet und den Beginn und das Ende des ausgeübten Drucks angibt, der Kreis 2 (39) im Rand des Rückstoßzylinders 2 (28) und der schwarze Gummi (40) ist in den Kreis 2 (39) integriert, der hintere Teil (41) im Rand des Zylinders (10) und der kleine Zylinder 1 (42), der von der Mitte des hinteren Teils (41) ausgeht. Die Kolbenabdeckung (9) ist mit dem Rückstoßzylinder 2 (28) verbunden,

3. HVS- und Pap-Abstrich-Testsystem, das selektiv verschiedene Spitzen (35, 47) und Mittel (9+28, 10+15+16+18) zur Betätigung der Probenentnahme nach Anspruch 1 verwendet, wobei das System so ausgelegt ist, dass es von der Patientin selbst oder vom Arzt verwendet werden kann, wobei der Probensammler mit einer Bürstenspitze (47) versehen ist, wobei der Flügel (43) sicherstellt, dass die Bürstenspitze (47) eine vollständige Tour im Rand der Spule (31) dreht, wobei der kleine Konus (44) den Durchgang des Rückstoßzylinders 2 (28) durch das Loch in einer Richtung 2 (29) ermöglicht, wobei der kleine Zylinder 2 (45) die weitere Bewegung der Bürstenspitze (47) in der Kante des Flügels (43) gewährleistet, wobei der Ring 7 (48) den Spitzenhalter (26) fixiert und das System verriegelt, Erleichterung der Probenentnahme und Sicherstellung der Drehung in einer Richtung, die Kopfstange 2 (46) ist mit dem Ring 7 (48) verbunden, die Bürste (47) befindet sich am Rand der Kopfstange 2 (46), eine längere Feder 2 (22) ist zwischen dem Ring 2a (7) und dem Ring 4 (24) vorhanden, damit sich die Bürste (47) weiter drehen kann, eine längere Feder 3 (27), die im inneren des Innenzylinders 2 (23) vorhanden ist, damit sich die Bürste (47) stärker drehen kann, der Spitzenhalter (26), der an einem Ende geschlossen ist, damit er an der Bürste (47) vorbeigeführt werden kann, die spiralförmige und im Querschnitt quer verlaufende Borste (49) der Bürste (47), wobei die scharfe Kante (50) der Borste (49) der Bürste (47) sicherstellt, dass die Zelle leicht aus dem Gebärmutterhals entnommen wird.

4. HVS- und Pap-Abstrich-Testsystem, das selektiv verschiedene Spitzen (35, 47) und Mittel (9+28, 10+15+16+18) zur Betätigung der Probenentnahme nach Anspruch 1 verwendet, wobei das System so ausgelegt ist, dass es von der Patientin und vom Arzt verwendet werden kann, wobei der Probensammler eine Baumwollspitze (35) und eine Bürstenspitze (47) hat, wobei die Schutzhülle (11) auf den Kopf (2) aufsetzbar ist und aus sehr dünnem, weichem Material hergestellt ist, das den Kopf umhüllt, die rutschig ist und eine Länge von 4 cm hat, die gebogene äußere Randseite (13) und die schräge innere Randseite (14) in der Randseite (12) der Schutzhülle (11).

## Revendications

1. Un système de test HVS et Pap smear utilisant de manière sélective différents embouts (35, 47) et des instruments (9+28, 10+15+16+18) pour mobiliser l'échantillonnage comprend ce qui suit : Un cylindre extérieur (1), une tête (2) à la première extrémité du cylindre extérieur (1), une face intérieure (3) de la tête (2), une face terminale (4) de la tête (2), une première bague (5) sur la surface extérieure et proche de la deuxième extrémité opposée à la première extrémité du cylindre extérieur (1), une extrémité en spirale (6) à la deuxième extrémité du cylindre extérieur (1), une deuxième bague (7) à l'intérieur du cylindre extérieur (1), un cylindre intérieur 1 (8), un chapeau de piston (9), un cylindre (10), un capuchon de protection (11) pouvant être monté sur la tête (2), une partie d'extrémité (12) du capuchon de protection (11), un côté d'extrémité extérieur (13) de la partie d'extrémité (12), un côté d'extrémité intérieur (14) de la partie d'extrémité (12), un tube flexible (15) raccordé au cylindre (10), un tube médical (16) raccordé au tube flexible (15), un ressort 1 (17) disposé dans le tube médical (16), un cylindre de recul 1 (18) coulissant dans le tube médical (16) pour appliquer une pression sur le ressort 1 (17), une ligne 1 (19. a) sur le tube médical (16), une ligne 2 (19. b) ; un anneau 3 (20) dans le cylindre extérieur (1) ; un anneau 2b (21) dans le cylindre extérieur (1) ; un ressort 2 (22) ; un cylindre intérieur 2 (23) dans le cylindre intérieur 1 (8) ; un anneau 4 (24) ; un trou 1 (25) à l'extrémité du cylindre intérieur (23) ; un support d'extrémité (26) ; un ressort 3 (27), un galet de rappel 2 (28) coulissant dans le cylindre intérieur 2 (23), un trou 2 (29), un cercle 1 (30) dans la partie arrière du galet de rappel 2 (28), une bobine (31), un anneau 5 (32), un anneau 6 (33), une tige de tête 1 (34) maintenue par le support d'extrémité (26), un coton (35) sur le bord de la tige de tête 1 (34), une cavité (36) dans le support d'extrémité (26), une marque de véracité (37) sur la tige de tête 1 (34), un cône (38) sur le cylindre de recul 2 (28), un cercle 2 (39) placé dans le cylindre (10), un pneu noir (40) intégré au cercle 2 (39), une partie arrière (41) sur le bord du cylindre (10), un petit cylindre 1 (42) traversant le centre de la partie arrière (41) et pouvant être relié au tube flexible (15), une aile (43) sur la bobine (31), un petit cône (44), un petit cylindre 2 (45) à l'extrémité de la bobine (31), une barre de tête 2 (46) maintenue sélectivement par le support d'extrémité (26) lorsque la barre de tête 1 (34) n'est pas connectée, une brosse (47) sur le bord de la barre de tête 2 (46), un anneau 7 (48) sur l'autre bord de la barre de tête 2 (46), un poil (49) de la brosse (47), un bord tranchant (50) du poil (49) et un trou 3 (51) dans le bouchon du piston (9), Ici l'appareil d'essai est équipé sélectivement d'un coton (35) ou d'une brosse (47), de sorte que différents embouts peuvent être utilisés de manière sélective Ici le cylindre extérieur (1) a un diamètre compris entre 1,6 et 2,4 cm et une longueur comprise entre 10 et 14 cm, le bouchon (2) se croise longitudinalement à l'avant et est adapté pour s'ouvrir en forme de rose,
La face intérieure (3) du bouchon (2) est fine et plate pour faciliter l'ouverture du bouchon (2); l'extrémité incurvée et épaisse (4) est destinée à l'inspection,
L'Anneau 1 (5) a une épaisseur de 1 à 1,2 cm, l'extrémité en spirale (6) permet d'intégrer sélectivement le chapeau de piston (9) ou le cylindre (10) dans le cylindre extérieur (1), ce qui permet d'utiliser sélectivement différents moyens pour actionner l'échantillonnage,
L'Anneau 2a (7) et L'Anneau 2b (21) relient l'extrémité spirale (6) au chapeau de piston (9) et permettent au cylindre intérieur 1 (8) de se déplacer, le cylindre intérieur 1 (8) se déplaçant librement dans le cylindre extérieur 1 (1),
L'Anneau 3 (20) comporte une saillie de 1 mm sur son bord pour empêcher le déplacement du cylindre intérieur 1 (8),
Le ressort 2 (22) est situé entre l'anneau 2a (7) et l'anneau 4 (24), le rouleau intérieur 2 (23) est fermé par l'avant dans le rouleau intérieur 1 (8),
L'Anneau 4 (24) s'insère sur le ressort 2 (22) au bord du cylindre intérieur 2 (23),
Le trou1 (25) est située au centre, ce qui permet au support d'extrémité (26) de pénétrer dans le bord de la bobine (31),
Insérez la tête de coton (35) en plaçant le support d'extrémité (26) avec l'extrémité ouverte,,
Le ressort 3 (27) se trouve dans le cylindre intérieur 2 (23),
Le cylindre de recul 2 (28) assure la pression,
Le trou 2 (29) est carré, ce qui permet au cylindre de recul 2 (28) d'être connecté à la bobine 31 à l'avant,
Le cercle 1 (30) facilite la poussée du pouce sur la partie arrière du cylindre de recul 2 (28),
Le cône (38) empêche le bouchon du piston (9) de sortir,
Le trou 3 (51) permet d'intégrer le chapeau de piston (9) au cylindre de recul 2 (28),
La bobine (31) est pliée dans la tête carrée (2) à l'intérieur du cylindre de recul 2 (28),
L'Anneau 5 (32) se trouve entre la bobine (31) et le support d'extrémité (26),
L'Anneau 6 (33) fixe le support d'extrémité (26),
La barre de buse 1 (34) est en forme de cure-dent, l'espace vide (36) permet de contrôler le mouvement de la barre de buse 1 (34) pour l'échantillonnage lorsqu'une pression est appliquée dans le porte-bouton (26), et la marque de précision (37) indique qu'un échantillon a été prélevé après la disparition de la ligne rouge sur la marque de précision (37) par le contact de l'appareil avec le bord lorsqu'il est poussé vers l'espace vide (36) après l'application d'une pression.

2. Le système de test HVS et Pap smear utilisant de manière sélective différents embouts (35, 47) et des moyens (9+28, 10+15+16+18) pour activer l'échantillonnage, tel que revendiqué dans la revendication 1, où l'appareil est conçu pour être utilisé par un patient, et où le système de test comporte un embout en coton (37) ou un embout brossé (47), et où le cylindre (10) est intégré au cylindre extérieur (1), le tube flexible (15) permet un mouvement entre le cylindre (10) et un tube médical (16) d'une longueur comprise entre 25 cm et 40 cm relié à un petit cylindre 1 (42), le tube médical (16) étant relié au tube flexible (15) utilisé pour l'injection, le ressort 1 (17) permettant à l'appareil de test de revenir à une position antérieure après l'application de la pression, le cylindre de recul 1 (18) permettant l'application de la pression, une ligne 1 (19.a) et une ligne 2 (19.b) vérifiant que l'échantillon est correctement prélevé lorsqu'une pression est appliquée au cylindre de recul 1 (18), un cercle 2 (39) au bord du cylindre de recul 2 (28) et un caoutchouc noir (40) intégré au cercle 2 (39) déterminant le début et la fin de la pression appliquée, une partie arrière (41) au bord du cylindre (10) et un petit cylindre 1 (42) traversant le centre de la partie arrière (41)."

3. "Le système de test HVS et Pap smear utilisant de manière sélective différents embouts (35, 47) et des moyens (9+28, 10+15+16+18) pour activer l'échantillonnage, tel que revendiqué dans la revendication 1, où le système est conçu pour être utilisé par le patient lui-même ou par un médecin, où l'embout préleveur est à brosse (47), où l'aile (43) de la brosse (47) permet à la brosse de faire un tour complet autour du bord de la bobine (31), où le petit cône (44) permet au petit cylindre de recul (28) de passer dans un sens (29) à travers le trou, où le petit cylindre 2 (45) permet un mouvement supplémentaire de l'aile (43) de la brosse (47) sur le bord, où l'anneau 7 (48) verrouille le porte-embout (26) en fixant le système, où la tige de tête 2 (46) intégrée à l'anneau 7 (48) facilite la procédure de prélèvement en permettant une rotation dans une seule direction, où la brosse (47) sur le bord de la tige de tête 2 (46), où un ressort 2 (22) plus long entre l'anneau 2a (7) et l'anneau 4 (24) permet une rotation supplémentaire de la brosse (47), où un ressort 3 (27) plus long à l'intérieur du cylindre intérieur 2 (23) permet une rotation supplémentaire de la brosse (47), où un embout fermé permet le passage de la brosse (47), où la brosse en forme de spirale et en section transversale (47) permet une prise facile des cellules cervicales."

4. Le système de test HVS et Pap smear utilisant de manière sélective différents embouts (35, 47) et des moyens (9+28, 10+15+16+18) pour activer l'échantillonnage, tel que revendiqué dans la revendication 1, où le système est conçu pour être utilisé par le patient et le médecin, où l'embout préleveur est en coton (35) et en brosse (47), où le capuchon protecteur (11) peut être monté sur la tête (2) et est fabriqué en un matériau très fin et doux qui entoure la tête, lisse et de 4 cm de long, avec un bord extérieur incurvé (13) et un bord intérieur incliné (14) sur le côté du bord du capuchon protecteur (11).
